# EUROPÄISCHE PATENTANMELDUNG

(11) **EP 1 426 023 A1**
(43) Veröffentlichungstag der Anmeldung: **09.06.2004**
(21) Anmeldenummer: 03405762.0
(22) Anmeldetag: 22.10.2003
(51) Int. Cl.: A61F 2/46

(54) **Verfahren, System und Computerprogrammprodukt zur Bestimmung von Implantationsparametern**

(30) Priorität: 22.11.2002 EP 02406011
(71) Anmelder: Sulzer Markets and Technology AG, 8401 Winterthur (CH)
(72) Erfinder: Zacharias, Thomas, Dr., 8400 Winterthur (CH); Eberlein, Robert, Dr., 8459 Volken (CH)
(74) Vertreter: Sulzer Management AG

(57) **Zusammenfassung**

Verfahren zur Bestimmung von Implantationsparametern für ein orthopädisches Implantat, in welchem Verfahren ausgehend von den Bilddaten einer Implantationsstelle Knochendaten eines zu behandelnden Knochens berechnet werden. Weiter umfasst das Verfahren eine biomechanische Analyse eines Systems bestehend aus dem zu behandelnden Knochen und dem Implantat. In der biomechanischen Analyse werden physikalische Systemgrössen berechnet, welche anschliessend mit gespeicherten Vergleichswerten verglichen werden. Der Vergleich ermöglicht eine Bewertung des durchgerechneten Systems. Die Daten des Systems stehen anschliessend als Implantationsparameter zur Weiterverarbeitung zur Verfügung.

## Beschreibung

Die Erfindung betrifft ein Verfahren und System zur Bestimmung von Implantationsparametern für ein orthopädisches Implantat gemäss den Merkmalen von Anspruch 1 und 9 und ein Computerprogrammprodukt zur Ausführung eines solchen Verfahrens.

Der künstliche Gelenkersatz nach pathologischen Veränderungen und Frakturen hat sich in den letzten Jahren als etablierte Behandlungsmethode durchgesetzt. Durch die Zunahme der degenerativen Gelenkserkrankungen und die gestiegene Lebenserwartung hat sich die Gelenkendoprothetik zu einem bedeutenden Zweig der Medizinaltechnik entwickelt. Ein Problem des künstlichen Gelenkersatzes betrifft die begrenzten Standzeiten der implantierten Endoprothesen. In den letzten Jahren wird deshalb verstärkt versucht, die Planung von Endoprothesenimplantationen derart zu verfeinern, dass sie exakter und reproduzierbarer umgesetzt werden können, um damit höhere Standzeiten zu erreichen und Planungsfehler zu vermeiden. Während sich in den Anfängen der Gelenkendoprothetik die Operationsplanung auf die visuelle Auswertung von Röntgenaufnahmen stützte, wurden in der Zwischenzeit Systeme und Methoden entwickelt, die eine dreidimensionale präoperative Planung auf der Basis von computertomografischen Daten ermöglichen. Dabei umfassen die entwickelten Systeme auch Fräsroboter, mit deren Hilfe die Planung exakt umgesetzt werden kann.

Ein grundlegendes Problem der präoperativen Planung betrifft die Ermittlung und insbesondere die Bewertung von Implantationsparametern, wie beispielsweise Typ, Form und Grösse des Implantates. Im Dokument [1] "Präoperative biomechanische Berechnung von Femur-Hüftenendoprothese-Systemen zur Ermittlung der individuellen Primärstabilität nach Roboterimplantationen", Dissertation von Thomas Zacharias vorgelegt am 29.09.2000 an der Universität Rostock, ISBN 3-8265-9153-4 wird ein biomechanisches Berechnungsverfahren für Femur-Hüftenendoprothese-Systeme beschrieben, welches im Rahmen von experimentellen und klinischen Untersuchungen validiert wurde. Das Dokument [1] erwähnt zwar den Einsatz eines biomechanischen Berechnungsverfahren bei der präoperativen Planung von Hüftendoprothesenimplantationen, ohne dass allerdings ein konkretes oder praxistaugliches Verfahren für die präoperative Planung oder die Bestimmung von Implantationsparametern offenbart wird.

Der Erfindung liegt die medizinaltechnische Aufgabenstellung zu Grunde, geeignete Planungshilfen zur Planung von orthopädischen Implantationen zur Verfügung zu stellen, insbesondere ein konkretes und praxistaugliches Verfahren und System zur Bestimmung von Implantationsparametern für ein orthopädisches Implantat und ein Computerprogrammprodukt zur Ausführung eines solchen Verfahrens. Weiter soll das Verfahren und das System eine optimierte präoperative Planung von Endoprothesenimplantationen ermöglichen, sowie eine exakte und reproduzierbare Umsetzung derselben.

Diese Aufgabe wird erfindungsgemäss durch das im unabhängigen Anspruch 1 definierte Verfahren und das in Anspruch 9 definierte System gelöst sowie durch das in Anspruch 8 definierte Computerprogrammprodukt.

Das erfindungsgemässe Verfahren zur Bestimmung von Implantationsparametern für ein orthopädisches Implantat umfasst das zur Verfügung Stellen von Bilddaten einer Implantationsstelle in einem Datenspeicher, das Berechnen von Knochendaten mindestens eines Knochens aus den Bilddaten sowie das zur Verfügung Stellen der Konstruktionsdaten mindestens eines orthopädischen Implantates in einem Datenspeicher, das Bestimmen der gegenseitigen Lage von Knochen und Implantat, insbesondere auf Grund der Geometrie, und das Speichern von Lagekoordinaten von Knochen und Implantat, das Bestimmen einer oder mehrerer Knochenkavitäten zur Verankerung und/oder Befestigung des Implantates im oder am Knochen und das Speichern der Kavitätsdaten. Weiter umfasst das erfindungsgemässe Verfahren eine biomechanische Analyse eines Systems umfassend mindestens den Knochen und das Implantat durch Berechnen einer oder mehrerer physikalischen Systemgrössen für vorgegebene Belastungen unter Verwendung von Konstruktionsdaten, Knochendaten, Lagekoordinaten und Kavitätsdaten und das Vergleichen der berechneten Systemgrössen mit gespeicherten Vergleichswerten sowie das zur Verfügung Stellen von Daten von mindestens einem der folgenden Implantationsparameter für die Weiterverarbeitung:
Implantattyp, Implantatform, Implantatgrösse und physikalische Eigenschaften des Implantates auf Grund der Konstruktionsdaten,
Knochenform und -grösse und physikalische Eigenschaften des Knochens auf Grund der Knochendaten,
Implantatposition und gegenseitige Lage von Knochen und Implantat auf Grund der Lagekoordinaten,
Kavitätsform und Kavitätsgrösse auf Grund der Kavitätsdaten, und berechnete Systemgrössen und Abweichungen der Systemgrössen von den Vergleichswerten auf Grund der biomechanischen Analyse.

Vorzugsweise umfasst das Verfahren zusätzlich das ein- oder mehrmalige Wiederholen des erfindungsgemässen Verfahrens mit geändertem Implantat und/oder geändertem System, insbesondere falls die berechneten Systemgrössen bezüglich der Vergleichswerte ein vorgegebenes Kriterium erfüllen.

In einer bevorzugten Ausführungsform umfasst das Verfahren zusätzlich das Berechnen von Gewebedaten von an den Knochen angrenzenden Weichgeweben aus den Bilddaten und die Verwendung der Gewebedaten der Weichgewebe bei der biomechanischen Analyse.

Vorteilhafterweise ist das Verfahren sowohl für zementierte als auch für zementfreie Verankerung des Implantates im Knochen einsetzbar. Vorzugsweise umfasst das Verfahren zusätzlich die Aufnahme der Bilddaten unter Verwendung bildgebender Mittel und eines bildgebenden Verfahrens. Vorzugsweise umfasst das Verfahren zusätzlich das zur Verfügung Stellen von Implantationsparametern zur Verwendung in der Steuerung eines Fräsroboters und/oder zur Verwendung als Sollwerte in einem Navigationssystem für Operationen.

Vorzugsweise gehört der Knochen, zu welchem die Knochendaten berechnet werden, zu einem der folgenden Skelettteile: Schultergelenk, Ellenbogengelenk, Hüftgelenk, Kniegelenk, Fussgelenk oder Wirbelsäule.

In einer weiteren bevorzugten Ausführungsform umfasst das Verfahren mindestens eines der folgenden Merkmale:
- dass die Bilddaten der Implantationsstelle mittels eines der folgenden bildgebenden Verfahren aufgenommen wurden:
   Röntgenverfahren, insbesondere computertomografische (CT-) Verfahren, sowie Ultraschall- (US-) Verfahren und kernspintomografische (MR-, NMR-) Verfahren;
- dass bei der Berechnung der Knochendaten mindestens Daten von einer der folgenden Grössen berechnet werden:
   Knochenform, Knochengrösse, Knochendichte und Materialkennwerte des Knochens;
- dass die Konstruktionsdaten des Implantates mindestens Daten zu einer der folgenden Eigenschaften umfassen:
   Implantattyp, Implantatform, Implantatgrösse und physikalische Eigenschaften des Implantates;
- dass die gegenseitige Lage von Knochen und Implantat auf Grund mindestens einer der folgenden Angaben bestimmt wird:
   Geometrie von Knochen und Implantat, früher bestimmte Lagekoordinaten und Resultate einer oder mehrerer vorangegangener biomechanischer Analysen;
- dass die Knochenkavität zur Verankerung und/oder Befestigung des Implantates im oder am Knochen auf Grund mindestens einer der folgenden Angaben bestimmt wird:
   Geometrie von Knochen und Implantat, Lagekoordinaten, Art der Verankerung und/oder Befestigung des Implantates im oder am Knochen, früher bestimmte Kavitätsdaten und Resultate einer oder mehrerer vorangegangener biomechanischer Analysen;
- dass die biomechanische Analyse folgende Schritte umfasst:
   Festlegung eines Berechnungsmodells, insbesondere eines Finite-Elemente-Modells;
   Festlegung von Modellparametern, insbesondere der Netzdichte;
   Zuweisung von physikalischen Eigenschaften, insbesondere von Materialkennwerten wie z.B. Elastizitätskennwerten, auf Grund der Knochendaten und Konstruktionsdaten;
   Zuweisung der Interfaceeigenschaften von Knochen und Implantat auf Grund der Art der Verankerung;
   Festlegung der Belastung, insbesondere Zuweisung eines Körpergewichtes und Festlegung des Belastungsfalles, beispielsweise Einbeinstand oder Treppensteigen;
   Berechnung mindestens einer der folgenden physikalischen Systemgrössen unter Verwendung von Konstruktionsdaten, Knochendaten, Lagekoordinaten und Kavitätsdaten: Dehnung im Knochen und im Implantat, mechanische Spannungen im Knochen und im Implantat, Relativverschiebung zwischen Implantat und Kochen, Temperaturerhöhung im Knochen;
- dass die berechneten Systemgrössen mit gespeicherten Grenzwerten und/oder mit gespeicherten Systemgrössen aus vorangegangenen biomechanischen Analysen verglichen werden.

Weiter umfasst die Erfindung ein Computerprogrammprodukt zur Ausführung der oben stehenden Verfahren auf einem Computer.

Weiter umfasst die Erfindung ein System zur Ausführung der oben stehenden Verfahren, insbesondere ein System mit einer oder mehreren Datenbanken, in welchen Konstruktionsdaten von orthopädischen Implantaten und/oder Vergleichs- und Erfahrungswerte von physikalischen Systemgrössen gespeichert sind, die im genannten Verfahren benötigt werden. Vorzugsweise enthält das System einen Fräsroboter und/oder eine Verbindung zu einer Steuereinheit eines Fräsroboters.

Mittels des erfindungsgemässen Verfahrens ist es möglich, die Implantationsparameter so zu optimieren, dass ein frühzeitiges Implantatversagen in Folge mangelnder Belastbarkeit, beispielsweise durch eine Implantatlockerung, vermieden werden kann. Die Verwendung derart optimierter Implantationsparameter als Sollwerte in Navigationssystemen für Operationen oder in der Steuerung von Fräsrobotern erlaubt es, Implantationen von Prothesen exakt und reproduzierbar auszuführen. Weitere Vorteile ergeben sich, wenn auch die den Knochen umgebenden Weichteile in die Bestimmung der Implantationsparameter einbezogen werden. Damit ist es möglich, die Implantationsparameter für ganze Bewegungsabläufe zu optimieren und nicht nur für einzelne Belastungsfälle.

Weitere vorteilhafte Ausführungsformen gehen aus den abhängigen Ansprüchen und der Zeichnung hervor.

Im Folgenden wird die Erfindung an Hand der Ausführungsbeispiele und an Hand der Zeichnung näher erläutert. Es zeigen:
- Fig. 1: ein Flussdiagramm eines Ausführungsbeispiel zum erfindungsgemässen Verfahren; und
- Fig. 2: eine schematische Darstellung eines Ausführungsbeispiels eines erfindungsgemässen System.

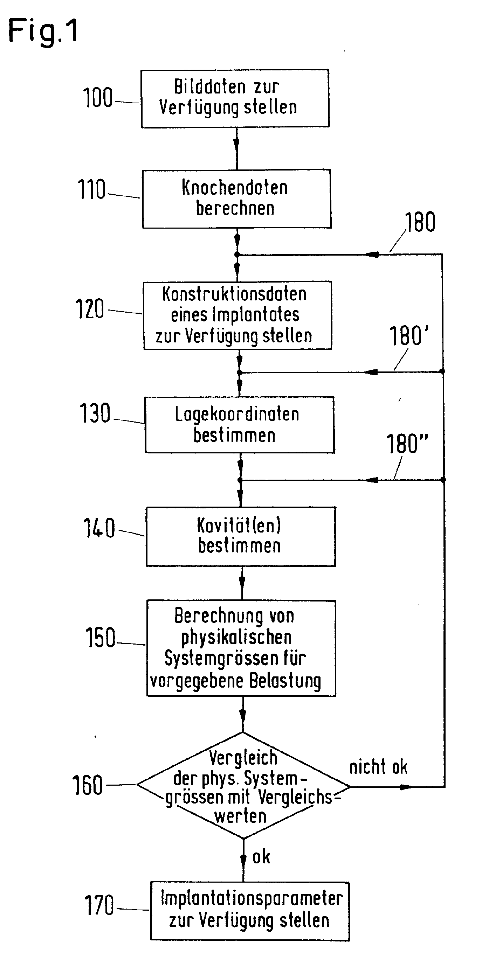

Fig. 1 zeigt ein Flussdiagramm eines Ausführungsbeispiels zum erfindungsgemässen Verfahren, In Fig. 1 bezeichnet die Bezugsnummer 100 einen ersten Verfahrensschritt, in welchem Bilddaten einer Implantationsstelle, beispielsweise eines degenerierten Gelenkes mit angrenzenden Bereichen, zur Verfügung gestellt werden. Typische Implantationsstellen sind z.B. ein Schultergelenk, Ellenbogengelenk, Hüftgelenk, Kniegelenk, oder Fussgelenk oder Teile der Wirbelsäule. Grundsätzlich ist das erfindungsgemässe Verfahren für alle Arten von orthopädischen Prothesen und/oder orthopädischen Implantaten anwendbar. Zweckmässigerweise werden die Bilddaten mittels eines bildgebenden Verfahrens, beispielsweise eines Röntgenverfahrens, z.B. computertomografischen (CT-) Verfahrens, oder eines Ultraschall- (US-) Verfahrens, kernspintomografisches (MR-, NMR-) Verfahrens, oder ähnlichen Verfahrens und mittels einer entsprechenden Anlage aufgenommen und in einem Datenspeicher gespeichert. In einem nächsten Schritt 110 werden aus den Bilddaten mittels Bildverarbeitung Knochendaten mindestens eines Knochens bestimmt. Vorteilhafterweise umfasst die Bildverarbeitung die Berechnung der Knochengeometrie, d.h. dessen Form und Grösse, und/oder die Berechnung der Knochendichte und/oder die Berechnung von Materialkennwerten, insbesondere Elastizitätskennwerten des Knochens.

In einem weiteren Schritt 120 werden in einem Datenspeicher Konstruktionsdaten mindestens eines Implantates, insbesondere eines orthopädischen Implantates, zur Verfügung gestellt. Die Konstruktionsdaten können den Implantattyp, die Implantatgrösse, die Implantatform oder physikalische Eigenschaften des Implantates umfassen. Zweckmässigerweise werden Konstruktionsdaten von mehreren Implantaten in einer Datenbank gespeichert, von welcher Konstruktionsdaten bei Bedarf abgerufen werden können. Die abgerufenen Konstruktionsdaten können anschliessend lokal zwischengespeichert werden. Die Auswahl eines Implantates aus einer Mehrzahl von Implantaten kann automatisch auf Grund geeigneter Kriterien erfolgen, beispielsweise auf Grund von geometrischen Kriterien unter Einbezug der in Schritt 110 berechneten Knochengeometrie und/oder auf Grund von Vorgaben, z.B. Vorgaben bezüglich korrektiver Massnahmen und/oder auf Grund von Festigkeitskriterien, z.B. unter Einbezug der Resultate vorangegangener biomechanischer Analysen, die in einem der folgenden Abschnitte näher erläutert werden. Es ist jedoch auch möglich, dass die Auswahl des Implantates interaktiv durch einen Orthopäden vorgenommen wird. Vorteilhafterweise werden die Bilddaten der Implantationsstelle beziehungsweise die Knochendaten gemeinsam mit den Konstruktionsdaten, insbesondere der Implantatform und -grösse auf einem Ausgabegerät dargestellt.

In einem weiteren Schritt 130 wird die gegenseitige Lage von Knochen und Implantat bestimmt. Die gegenseitige Lage kann automatisch auf Grund geeigneter Kriterien bestimmt werden, beispielsweise auf Grund von geometrischen Kriterien und/oder auf Grund von Vorgaben und/oder auf Grund von Festigkeitskriterien analog zur Implantatauswahl in Schritt 120. Es ist jedoch auch möglich, dass die gegenseitige Lage interaktiv durch einen Orthopäden bestimmt wird. Die gegenseitige Lage von Knochen und Implantat ist durch Lagekoordinaten festgelegt, die abgespeichert werden. Zweckmässigerweise werden die Knochen- und Implantatgeometrie zusammen mit der oben bestimmten gegenseitigen Lage vor dem Speichern in ein gemeinsames Koordinatensystem transformiert.

In Schritt 140 werden abhängig von der Verankerung und/oder Befestigung des Implantates im oder am Knochen eine oder mehrere Knochenkavitäten bestimmt. Die Verankerung einer Endoprothese kann beispielsweise mittels Presspassung, auch "Press fit" genannt, erfolgen oder mittels Einzementieren der Prothese in einer Knochenkavität. Die Verankerung und/oder Befestigung kann vorgegeben werden oder bedarfsweise interaktiv von einem Orthopäden eingegeben werden. Bei gegebener Verankerung können die für die Implantation benötigten Knochenkavitäten automatisch bestimmt und die entsprechenden Kavitätsdaten abgespeichert werden.

In Schritt 150 wird eine biomechanische Analyse eines Systems umfassend mindestens den Knochen und das Implantat durchgeführt. Die biomechanische Analyse umfasst typischerweise:
Festlegen eines Berechnungsmodels, insbesondere eines Finite-Elemente-Modells;
Festlegen von Modellparametern, insbesondere der Netzdichte;
Zuweisung von physikalischen Eigenschaften, insbesondere von Materialkennwerten, auf Grund der Knochendaten und Konstruktionsdaten;
Zuweisung der Interfaceeigenschaften von Knochen und Implantat auf Grund der Art der Verankerung und/oder Befestigung;
Festlegung der Belastung, insbesondere Zuweisung eines Körpergewichtes und Festlegung des Belastungsfalles, wie z.B. Einbeinstand oder Treppensteigen;
Berechnung mindestens einer physikalischen Systemgrösse, wie z.B. der Dehnung im Knochen oder im Implantat, der mechanische Spannungen im Knochen oder im Implantat, der Relativverschiebung zwischen Implantat und Kochen, der Temperaturerhöhung im Knochen u.a.m., für die festgelegte Belastung unter Verwendung von Konstruktionsdaten, Knochendaten, Lagekoordinaten und Kavitätsdaten.

Die berechneten Systemgrössen werden in einem weiteren Schritt 160 mit gespeicherten Vergleichswerten verglichen. Die Vergleichswerte können beispielsweise Maximal- und/oder Minimalwerte für die entsprechenden Systemgrössen umfassen oder anzustrebende Erfahrungswerte. Vorteilhafterweise werden die Vergleichswerte in einer Datenbank gespeichert, welche mit Werten aus der klinischen Praxis nachgeführt und ergänzt werden kann. Die Datenbank kann auch als Expertensystem organisiert sein. Die gespeicherten Vergleichswerte können auch Systemgrössen aus einer oder mehreren vorangegangenen biomechanischen Analysen umfassen. Dies ermöglicht eine Optimierung von Implantationsparametern, indem z.B. die zu optimierenden Implantationsparameter variiert werden und anschliessend die Systemgrössen aus den zugehörigen biomechanischen Analysen miteinander verglichen werden.

In Schritt 170 werden Daten von mindestens einem der folgenden Implantationsparameter für die Weiterverarbeitung zur Verfügung gestellt:
Implantattyp, Implantatform, Implantatgrösse und physikalische Eigenschaften des Implantates auf Grund der Konstruktionsdaten,
Knochenform und -grösse und physikalische Eigenschaften des Knochens auf Grund der Knochendaten,
Implantatposition und gegenseitige Lage von Knochen und Implantat auf Grund der Lagekoordinaten,
Kavitätsform und Kavitätsgrösse auf Grund der Kavitätsdaten, und berechnete Systemgrössen und Abweichungen der Systemgrössen von den Vergleichswerten auf Grund der biomechanischen Analyse.

In einer Ausführungsvariante umfasst die Weiterverarbeitung die Anzeige der in Schritt 170 zur Verfügung gestellten Implantationsparameter und/oder deren Verwendung in der Steuerung eines Fräsroboters und/oder deren Verwendung als Sollwerte in einem Navigationssystem für Operationen und/oder deren Verwendung in der Erstellung von Steuerprogrammen für einen Fräsroboter oder für ein Navigationssystem für Operationen.

In einer weiteren Ausführungsvariante werden zumindest Teile des oben beschriebenen Verfahrens mit einem geänderten Implantat und/oder mit einem geänderten System wiederholt, typischerweise wenn die berechneten Systemgrössen in dem in Schritt 160 durchgeführten Vergleich bezüglich der Vergleichswerte ein vorgegebenes Kriterium erfüllen. Ein solches Kriterium kann z.B. das Überschreiten eines Grenzwertes, wie beispielsweise der gespeicherten Maximaldehnung im Knochen, sein. Die entsprechenden Verbindungen für die Wiederholung sind in dem in Fig. 1 dargestellten Flussdiagramm mit den Bezugszeichen 180, 180' und 180" bezeichnet. Das geänderte Implantat und/oder System kann automatisch, beispielsweise auf Grund der berechneten Systemgrössen, ermittelt werden oder auch interaktiv durch einen Orthopäden eingegeben werden.

In einer bevorzugten Ausführungsform des Verfahrens werden in Schritt 110 aus den Bilddaten mittels Bildverarbeitung zusätzlich Gewebedaten von an den Knochen angrenzenden Weichgeweben, wie beispielsweise Bänder, Knorpel u.a.m. bestimmt. Vorteilhafterweise umfasst die Bildverarbeitung die Berechnung der Geometrie der Weichgewebe, d.h. deren Form und Grösse, und/oder die Berechnung der Dichte der Weichgewebe und/oder die Berechnung von Materialkennwerten, insbesondere Elastizitätskennwerten der Weichgewebe. Vorteilhafterweise umfasst das zu analysierende System in der biomechanischen Analyse in Schritt 150 zusätzlich zum Knochen und Implantat auch Weichgewebe. Ohne Weichgewebe wirken die Belastungskräfte normalerweise punktförmig auf den Knochen, wobei die Lage der Ansatzpunkte für die Kräfte von der momentanen Knochenstellung und -belastung abhängt. Bei Einbezug von Weichgeweben in das zu analysierende System greifen die Kräfte zum Teil an den Weichgeweben an, was die Berechnung von physikalischen Systemgrössen für ganze Bewegungsabläufe wesentlich einfacher und genauer macht.

Das Ausführungsbeispiel zur vorliegenden Erfindung umfasst auch ein Computerprogrammprodukt, insbesondere ein Computerprogramm auf einer Festplatte oder auf einem Datenträger, wie beispielsweise auf einer Diskette, CD, DVD oder einem ähnlichen Datenträger zur Ausführung des erfindungsgemässen Verfahrens auf einem Computer.

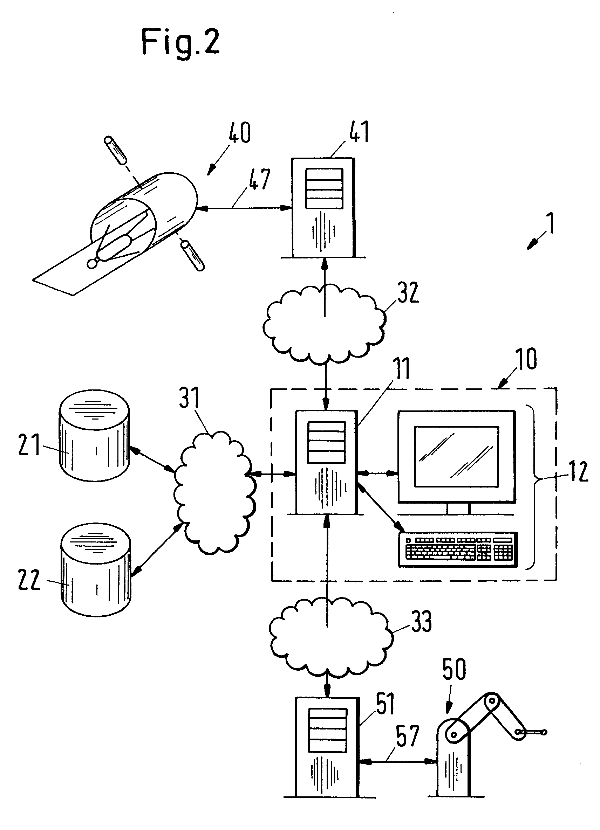

Fig. 2 zeigt eine schematische Darstellung eines Ausführungsbeispiels eines Systems zur Ausführung des erfindungsgemässen Verfahrens. In Fig. 2 bezeichnet die Bezugsnummer 10 einen Computer umfassend eine Recheneinheit 11 und eine Ein- und Ausgabeeinheit 12 zur Anzeige von Ausgabedaten, wie z.B. Bilddaten, Konstruktionsdaten, physikalischen Systemgrössen und Implantationsparametern, und zur interaktiven Eingabe von Daten. Die Recheneinheit 11 umfasst einen Programmspeicher, in dem ein Computerprogramm zur Ausführung des weiter oben beschriebenen Verfahrens gespeichert ist. Der Computer 10 ist über eine Datenverbindung 31, beispielsweise über eine Wählverbindung, über ein Local Area Network (LAN) oder Wide Area Network (WAN), über Intranet oder Internet oder über eine ähnliche Verbindung, mit einer oder mehreren Datenbanken 21 und 22 verbunden, in denen Konstruktionsdaten von orthopädischen Implantaten beziehungsweise Vergleichswerte für physikalische Systemgrössen für ein System umfassend mindestens einen Knochen und ein Implantat gespeichert sind. Selbstverständlich können die Datenbanken 21 und 22 auch auf einem transportablen Datenträger gespeichert sein oder direkt in der Recheneinheit 11. In einer Ausführungsvariante ist der Computer 10 über eine Datenverbindung 32, die beispielsweise über eine Direktverbindung, Wählverbindung, oder über eine LAN-, WAN-, Intranet-, Internet- oder ähnliche Verbindung realisiert sein kann, mit einem Aufnahmegerät 40 für Bilddaten einer Implantationsstelle verbunden. Als Aufnahmegerät kann beispielsweise ein Röntgengerät, insbesondere ein Computertomograf, oder ein Kernspintomograf, Ultraschallgerät oder ähnliches bildgebendes Gerät vorgesehen sein. Selbstverständlich ist es auch möglich, Mittel vorzusehen, um Bilddaten eines bildgebenden Gerätes auf einem transportablen Datenträger zu speichern und durch die Recheneinheit 11 vom transportablen Datenträger abzurufen. In einer weiteren Ausführungsvariante ist der Computer 10 über eine Datenverbindung 33, die beispielsweise über eine Direktverbindung, Wählverbindung, oder über eine LAN-, WAN-, Intranet-, Internet- oder ähnliche Verbindung realisiert sein kann, mit einem Fräsroboter 50 verbunden, beziehungsweise mit einer Steuereinheit 51 des Fräsroboters, um die mit Hilfe des erfindungsgemässen Verfahrens bestimmten Implantationsparameter der Steuereinheit des Fräsroboters zu Verfügung zu stellen. In gleicher Weise ist es möglich, die mit Hilfe des erfindungsgemässen Verfahrens bestimmten Implantationsparameter über eine Datenverbindung einem Navigationssystem für Operationen oder einem anderen Hilfsmittel für Operationen zur Verfügung zu stellen. Anstelle der Datenverbindung 33 können Mittel vorgesehen sein, um Daten betreffend Implantationsparameter auf einem transportablen Datenträger zu speichern und durch die Steuereinheit 51 des Fräsroboters beziehungsweise durch das erwähnte Navigationssystem oder durch ein anderes Hilfsmittel für Operationen vom transportablen Datenträger abzurufen. Die Verwendung der zuvor bestimmten Implantationsparameter in der Steuerung des Fräsroboters, des Navigationssystems und/oder eines anderen Hilfsmittels für Operationen erlaubt eine exakte und reproduzierbare Umsetzung der geplanten Implantation.

## Patentansprüche

1. Verfahren zur Bestimmung von Implantationsparametern für ein orthopädisches Implantat umfassend
- zur Verfügung Stellen von Bilddaten einer Implantationsstelle in einem Datenspeicher;
- Berechnen von Knochendaten mindestens eines Knochens aus den Bilddaten;
- zur Verfügung Stellen der Konstruktionsdaten mindestens eines orthopädischen Implantates in einem Datenspeicher;
- Bestimmen der gegenseitigen Lage von Knochen und Implantat, insbesondere auf Grund der Geometrie, und Speichern von Lagekoordinaten von Knochen und Implantat;
- Bestimmen einer oder mehrerer Knochenkavitäten zur Verankerung und/oder Befestigung des Implantates im oder am Knochen und Speichern der Kavitätsdaten;
- biomechanische Analyse eines Systems umfassend mindestens den Knochen und das Implantat durch Berechnen einer oder mehrerer physikalischen Systemgrössen für vorgegebene Belastungen unter Verwendung von Konstruktionsdaten, Knochendaten, Lagekoordinaten und Kavitätsdaten;
- Vergleichen der berechneten Systemgrössen mit gespeicherten Vergleichswerten;
- Zur Verfügung Stellen von Daten von mindestens einem der folgenden Implantationsparameter für die Weiterverarbeitung:
Implantattyp, Implantatform, Implantatgrösse und physikalische Eigenschaften des Implantates auf Grund der Konstruktionsdaten,
Knochenform und -grösse und physikalische Eigenschaften des Knochens auf Grund der Knochendaten,
Implantatposition und gegenseitige Lage von Knochen und Implantat auf Grund der Lagekoordinaten,
Kavitätsform und Kavitätsgrösse auf Grund der Kavitätsdaten, und berechnete Systemgrössen und Abweichungen der Systemgrössen von den Vergleichswerten auf Grund der biomechanischen Analyse.

2. Verfahren nach Anspruch 1 umfassend zusätzlich
- Wiederholen des Verfahrens mit geändertem Implantat und/oder geändertem System, insbesondere falls die berechneten Systemgrössen bezüglich der Vergleichswerte ein vorgegebenes Kriterium erfüllen.

3. Verfahren nach einem der Ansprüche 1 oder 2 umfassend zusätzlich
- Berechnen von Gewebedaten von an den Knochen angrenzenden Weichgeweben aus den Bilddaten; und
- Verwendung der Gewebedaten bei der biomechanischen Analyse.

4. Verfahren nach einem der vorangehenden Ansprüche, wobei das Verfahren sowohl für zementierte als auch für zementfreie Verankerung des Implantates im Knochen einsetzbar ist.

5. Verfahren nach einem der vorangehenden Ansprüche umfassend zusätzlich mindestens einen der folgenden Schritte:
- Aufnahme der Bilddaten unter Verwendung bildgebender Mittel und eines bildgebenden Verfahrens.
- zur Verfügung Stellen von Implantationsparametern zur Verwendung in der Steuerung eines Fräsroboters und/oder zur Verwendung als Sollwerte in einem Navigationssystem für Operationen.

6. Verfahren nach einem der vorangehenden Ansprüche, wobei der Knochen, zu welchem die Knochendaten berechnet werden, zu einem der folgenden Skelettteile gehört:
Schultergelenk, Ellenbogengelenk, Hüftgelenk, Kniegelenk, Fussgelenk oder Wirbelsäule.

7. Verfahren nach einem der vorangehenden Ansprüche umfassend mindestens eines der folgenden Merkmale:
- dass die Bilddaten der Implantationsstelle mittels eines der folgenden bildgebenden Verfahren aufgenommen wurden:
Röntgenverfahren, insbesondere computertomografische (CT-) Verfahren, oder Ultraschall- (US-) Verfahren oder kernspintomografische (MR-, NMR-) Verfahren;
- dass bei der Berechnung der Knochendaten mindestens Daten von einer der folgenden Grössen berechnet werden:
Knochenform, Knochengrösse, Knochendichte und Materialkennwerte, insbesondere Elastizitätskennwerte des Knochens;
- dass die Konstruktionsdaten des Implantates mindestens Daten zu einer der folgenden Eigenschaften umfassen:
Implantattyp, Implantatform, Implantatgrösse und physikalische Eigenschaften des Implantates;
- dass die gegenseitige Lage von Knochen und Implantat auf Grund mindestens einer der folgenden Angaben bestimmt wird:
Geometrie von Knochen und Implantat, früher bestimmte Lagekoordinaten und Resultate einer oder mehrerer vorangegangener biomechanischer Analysen;
- dass die Knochenkavitäten zur Verankerung und/oder Befestigung des Implantates im oder am Knochen auf Grund mindestens einer der folgenden Angaben bestimmt werden:
Geometrie von Knochen und Implantat, Lagekoordinaten, früher bestimmte Kavitätsdaten, Art der Verankerung des Implantates im Knochen und Resultate einer oder mehrerer vorangegangener biomechanischer Analysen;
- dass die biomechanische Analyse folgende Schritte umfasst:
Festlegung eines Berechnungsmodels, insbesondere eines Finite-Elemente-Modells;
Festlegung von Modellparametern, insbesondere der Netzdichte; Zuweisung von physikalischen Eigenschaften, insbesondere von Materialkennwerten, auf Grund der Knochendaten und Konstruktionsdaten;
Zuweisung der Interfaceeigenschaften von Knochen und Implantat auf Grund der Art der Verankerung;
Festlegung der Belastung, insbesondere Zuweisung eines Körpergewichtes und Festlegung des Belastungsfalles, beispielsweise Einbeinstand oder Treppensteigen;
Berechnung mindestens einer der folgenden physikalischen Systemgrössen unter Verwendung von Konstruktionsdaten, Knochendaten, Lagekoordinaten und Kavitätsdaten: Dehnung im Knochen und im Implantat, mechanische Spannungen im Knochen und im Implantat, Relativverschiebung zwischen Implantat und Kochen, Temperaturerhöhung im Knochen;
- dass die berechneten Systemgrössen mit gespeicherten Grenzwerten und/oder mit gespeicherten Systemgrössen aus vorangegangenen biomechanischen Analysen verglichen werden;

8. Computerprogrammprodukt zur Ausführung eines Verfahrens nach einem der vorangehenden Ansprüche auf einem Computer.

9. System zur Ausführung eines Verfahrens nach einem der Ansprüche 1 bis 7.

10. System nach Anspruch 9, wobei das System eine oder mehrere Datenbanken enthält, in welchen Konstruktionsdaten von orthopädischen Implantaten und/oder Vergleichs- und Erfahrungswerte von physikalischen Systemgrössen gespeichert sind, die im genannten Verfahren benötigt werden, und/oder wobei das System einen Fräsroboter und/oder eine Verbindung zu einer Steuereinheit eines Fräsroboters enthält.
